# EUROPEAN PATENT SPECIFICATION

(11) **EP 0 525 494 B1**
(45) Date of publication and mention of the grant of the patent: **12.10.1994**
(21) Application number: 92111910.3
(22) Date of filing: 13.07.1992
(51) Int. Cl.: C07H 15/04, C11D 1/66, A61K 7/00

(54) **Polyglucosides obtained from hydroxyalkylsorbitol**
Polyglukosiden aus Hydroxyalkylsorbit
Polyglucosides obtenus à partir d'hydroalkylsor-Bitol

(30) Priority: 29.07.1991 IT MI912104
(43) Date of publication of application: 03.02.1993
(73) Proprietor: AUSCHEM S.p.A., 20134 Milano (IT)
(72) Inventor: Fornara, Dario, I-28100 Novara (IT); Bernardi, Paolo, I-20090 Vimodrone (MI) (IT); Garlisi, Salvatore, I-13100 Vercelli (IT)
(74) Representative: Giambrocono, Alfonso, Dr. Ing.

(56) References cited:
- EP-A- 0 077 167
- DE-A- 1 155 771
- FR-A- 2 661 413
- US-A- 4 376 198

## Description

The present invention relates to polyglucosidic compounds having surface-active properties.

Fatty alcohol polyglucosidic compounds are known as surface-active agents.

We have now found some polyglucosidic surface-active agents different from those known in that the hydroxyalkylsorbitol group is bound to the polyglucosidic chain.

The hydroxyalkylsorbitol polyglucosides of the present invention have the general formula (I): 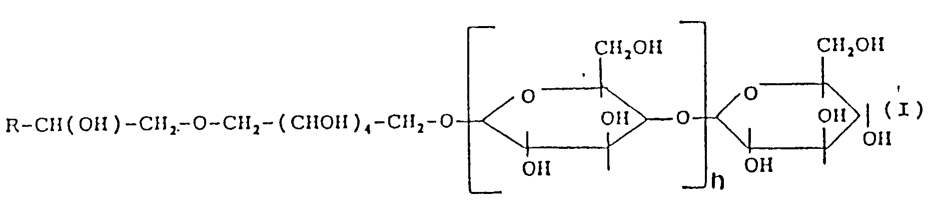
wherein:
- R is a C₆-C₂₀ alkyl, n is an integer from 0 to 20.

The products of general formula (I) where R = C₈-C₁₀ alkyl and n is comprised between 0 and 5 are preferred.

The products of formula (I) are prepared with a process comprising the hydroxyalkylsorbitol glucosidation at 120°C, at 60 mmHg residual and in the presence of acid catalysts.

The hydroxyalkylsorbitol polyglucosides of the present invention, show a set of features such as biodegradability, non toxicity, lack of irritant effects for the skin, good solubility in water combined with good detergent properties which make them particularly flexible and usable in the various fields e.g., the detergency in general, in the toilet and cosmetic field and in the textile industry.

It has been found that the hydroalkylsorbitol polyglucosides of the present invention are compatible with most of the known anionic, cationic, amphoteric and non-ionic surface-active agents, so they can be formulated with them.

For a better understanding of the possibility of the present invention the following examples are only given for an illustrative purpose.

### Example 1

### Preparation of the hydroxydecilsorbitol polyglucoside.

338 g of hydroxydecilsorbitol were introduced in a reactor, equipped with heating system, stirrer, thermometer, a refrigerating system connected with vacuum. It was heated at 80°C and 10.7 g of paratoluensulphonic acid and 0.8 g of hypophosphorus acid in a solution at 50% in water were introduced in the reactor.

The temperature was brought to 120°C and 180 g of anhydrous dextrose were charged in portions of 30 g each, at intervals of 20 minutes.

After 20 minutes from the last addition, the pressure was brought to athmospheric pressure and the temperature to 60°C.

The obtained mass was neutralized by adding 2.4 g of NaOH in a solution at 30% by weight and then cooled at room temperature.

A yellow-coloured liquid containing 40% by weight of water and 60% by weight of hydroxydecilsorbitol polyglucosidated, having an hydroxyl number of 1050, was obtained.

The diluted solutions of the obtained product were opalescent.

### Example 2

Example 1 was repeated but using hydroxyoctilsorbitol. It was prepared the hydroxyoctilsorbitol polyglucoside.

### Products characteristics

In tables 1 and 2 are reported the surface tension and the foaming power values of the products of examples 1 and 2, determined by the following methods.
The surface tension was measured at 20°C by du-Nouy method on solutions containing 0.1% by weight of surface-active agent; The foaming power was determined by Ross-Miles method at 2 concentrations.

**TABLE 1**

| SURFACE TENSION (dine/cm) (1 g/l a.s.) | |
|---|---|
| Example 1 | 26.5 |
| Example 2 | 27.4 |

**TABLE 2**

| FOAMING POWER BY ROSS-MILES METHOD AT 25°C (hard water: 30°F) | | | | |
|---|---|---|---|---|
| Concentration a.s. | 0.5 g/l | | 1 g/l | |
| after minutes | 0' | 5' | 0' | 5' |
| Example 1 | 30 | 25 | 50 | 45 |
| Example 2 | 60 | 55 | 80 | 75 |

## Claims (Claims for the following Contracting State(s): AT, BE, CH, DE, DK, FR, GB, IT, LI, LU, NL, SE)

1. Hydroxyalkylsorbitol polyglucosides of general formula (I): 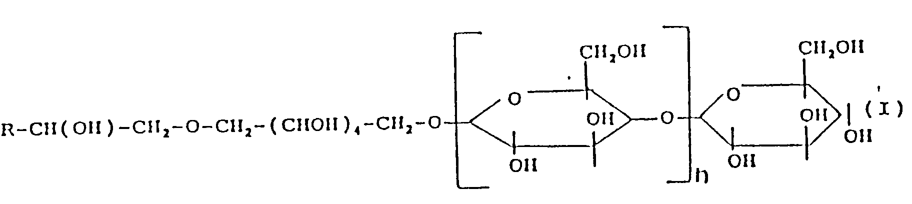 wherein:
R is a C₆-C₂₀ alkyl, n is an integer between 0 and 20.

2. Hydroxyalkylsorbitol polyglucosides, according to claim 1, wherein R is selected among C₈ - C₁₀ alkyls and n is a number between 0 and 5.

3. Cosmetic and detergent compositions containing one or more hydroxyalkylsorbitol polyglucosides, according to claims 1 and 2, alone or in admixture with other surface-active agents.

4. Use of the hydroxyalkylsorbitol polyglucosides, according to claims 1 and 2, as surface-active agents.

## Claims (Claims for the following Contracting State(s): ES, GR)

1. A process for the preparation of hydroxyalkylsorbitol polyglucosides of general formula (I): 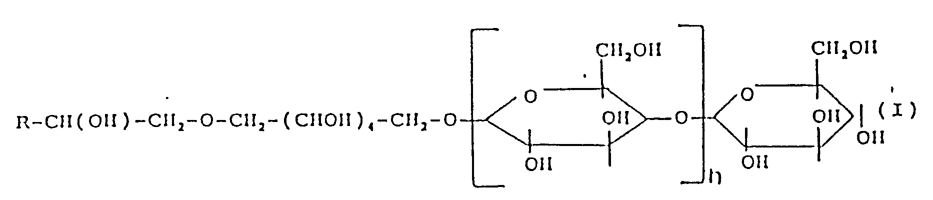 wherein:
R is a C₆-C₂₀ alkyl, n is an integer between 0 and 20, characterized by the hydroxyalkylsorbitol glycosidation at 120°C, at 60 mmHg residual and in the presence of acid catalyst.

2. A process according to claim 1, wherein R is selected among C₈-C₁₀ alkyls and n is a number between 0 and 5.

## Patentansprüche (Patentansprüche für folgende(n) Vertragsstaat(en): AT, BE, CH, DE, DK, FR, GB, IT, LI, LU, NL, SE)

1. Hydroxyalkylsorbitpolyglucoside der allgemeinen Formel (I): 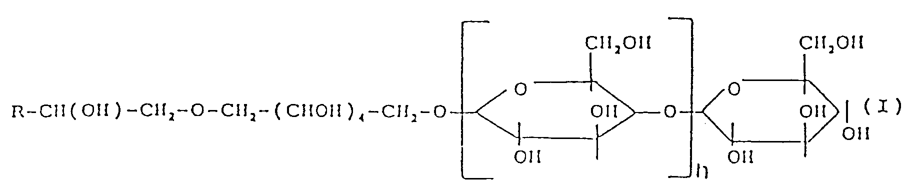 worin
R C₆-C₂₀-Alkyl bedeutet, n eine ganze Zahl zwischen 0 und 20 bedeutet.

2. Hydroxyalkylsorbitpolyglucoside nach Anspruch 1, dadurch **gekennzeichnet,** daß R ausgewählt wird aus C₈-C₁₀-Alkylen und n eine ganze Zahl zwischen 0 und 5 ist.

3. Kosmetische Zusammensetzungen und Detergens-Zusammensetzungen, dadurch **gekennzeichnet,** daß sie ein oder mehrere Hydroxyalkylsorbitpolyglucoside nach einem der Ansprüche 1 und 2 allein oder im Gemisch mit anderen grenzflächenaktiven Mitteln enthalten.

4. Verwendung der Hydroxyalkylsorbitpolyglucoside nach einem der Ansprüche 1 und 2 als grenzflächenaktive Mittel.

## Patentansprüche (Patentansprüche für folgende(n) Vertragsstaat(en): ES, GR)

1. Verfahren zur Herstellung von Hydroxyalkylsorbitpolyglucosiden der allgemeinen Formel (I): 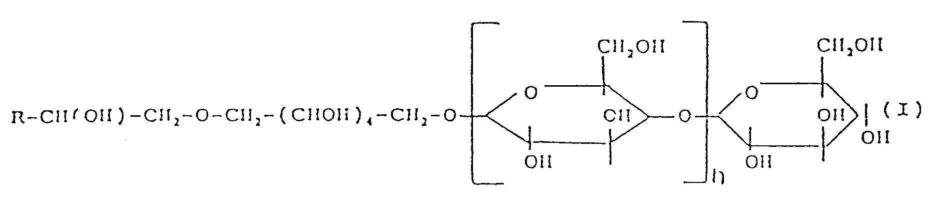 worin
R C₆-C₂₀-Alkyl bedeutet, n eine ganze Zahl zwischen 0 und 20 bedeutet, dadurch **gekennzeichnet,** daß eine Hydroxyalkylsorbit-Glycosidierung bei 120°C, bei 60 mmHg residual und in Anwesenheit eines sauren Katalysators durchgeführt wird.

2. Verfahren nach Anspruch 1, dadurch **gekennzeichnet,** daß R ausgewählt wird aus C₈-C₁₀-Alkylen und n eine ganze Zahl zwischen 0 und 5 ist.

## Revendications (Revendications pour l'(les) Etat(s) contractant(s) suivant(s): AT, BE, CH, DE, DK, FR, GB, IT, LI, LU, NL, SE)

1. Hydroxyalkylsorbitolpolyglucosides de formule générale (I) : 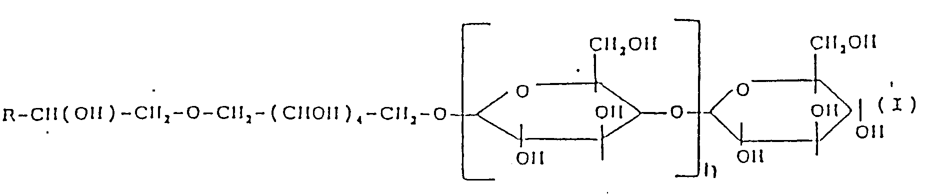 dans laquelle :
R est un radical alkyle en C₆-C₂₀ et n est un entier de 0 à 20.

2. Hydroxyalkylsorbitolpolyglucosides selon la revendication 1, dans lesquels R est choisi parmi les radicaux alkyle en C₈-C₁₀ et n est un entier de 0 à 5.

3. Compositions cosmétiques et détergentes contenant un ou plusieurs hydroxyalkylsorbitolpolyglucosides selon les revendications 1 et 2, seuls ou en mélange avec d'autres tensio-actifs.

4. Utilisation des hydroxyalkylsorbitolpolyglucosides selon les revendications 1 et 2 en tant qu'agents tensio-actifs.

## Revendications (Revendications pour l'(les) Etat(s) contractant(s) suivant(s): ES, GR)

1. Procédé pour préparer des hydroxyalkylsorbitolpolyglucosides de formule générale (I) : 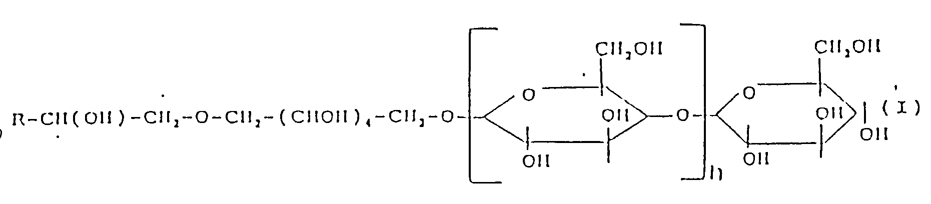 dans laquelle
R est un radical alkyle en C₆-C₂₀ et n est un entier de 0 à 20, caractérisé par la glycosidation d'un hydroxyalkylsorbitol à 120°C sous une pression résiduelle de 8 kPa (60 mmHg) et en présence d'un catalyseur acide.

2. Procédé selon la revendication 1, dans lequel R est choisi parmi les radicaux alkyle en C₈-C₁₀ et n est un entier de 0 à 5.
